# EUROPÄISCHE PATENTSCHRIFT

(11) **EP 0 507 234 B1**
(45) Veröffentlichungstag und Bekanntmachung des Hinweises auf die Patenterteilung: **16.08.2001**
(21) Anmeldenummer: 92105464.9
(22) Anmeldetag: 30.03.1992
(51) Int. Cl.: C12N 1/00, C08B 37/00

(54) **Verfahren zur Zellisolierung, isolierte Zellen (dsm 6314 und dsm 6418) und Verwendung isolierter Zellen zur Polysaccharidherstellung**
Method for cell isolation, isolated cells (dsm 6314 and dsm 618) and use of these isolated cells for preparing polysaccharids
Procédé pour l'isolation de cellules, cellules isolées (dsm 6314 et dsm 618) et utilisation de ces cellules isolées pour préparer des polysaccharides

(30) Priorität: 30.03.1991 DE 4110549
(43) Veröffentlichungstag der Anmeldung: 07.10.1992
(73) Patentinhaber: Gesellschaft für Biotechnologische Forschung mbH (GBF), D-38124 Braunschweig (DE)
(72) Erfinder: Deckwer, Wolf-Dieter, W-3300 Braunschweig (DE); Lobas, Detlef, W-3300 Braunschweig (DE); Schumpe, Adrian, W-3300 Braunschweig (DE)
(74) Vertreter: Boeters, Hans Dietrich, Dr.

(56) Entgegenhaltungen:
- EP-A- 0 012 552
- EP-A- 0 232 582
- EP-A- 0 353 145
- DE-A- 3 445 302
- PURE AND APPLIED CHEMISTRY Bd. 56, Nr. 7, 1984, OXFORD,GB Seiten 879 - 892 P.A. SANDFORD ET AL. 'Microbial polysaccharides: new products and their commercial applications'
- BIOTECHNOLOGY ABSTRACTS. DERWENT PUBLICATIONS LTD., LONDON, GB. ABSTRACT NO. 91-12497 L.C. BRITO ET AL. 'Effect of the aqueous soluble components of the immobilization matrix on ethanol and microbial exopolysaccharides production kappa-carrageenan support effect on ethanol, alginate and gellan gum production by immobilized Saccharomyces bayanus, Pseudomonas aeruginosa and Pseudomonas elodea'
- PATENT ABSTRACTS OF JAPAN vol. 12, no. 22 (C-470)(2869) 22. Januar 1988
- BIOTECHNOLOGY ABSTRACTS. DERWENT PUBLICATIONS LTD., LONDON, GB. ABSTRACT NO. 91-01612 T.A. GRINBERG ET AL. 'Microbial synthesis of exopolysaccharides in the culture liquid containing C1-C2-compounds - e.g. xantham gum, biosan gum, exopolysaccharide PS-84 and gellan gum production; methylotroph immobilization, review'
- JOURNAL OF APPLIED BACTERIOLOGY Bd. 62, Nr. 2, Februar 1987, OXFORD, GB. Seiten 147 - 150 A. ANSON ET AL. 'A bacterium yielding a polysaccharide with unusual properties'
- BIOTECHNOLOGY ABSTRACTS. DERWENT PUBLICATIONS LTD., LONDON, GB. ABSTRACT NO. 93-04622 D. LOBAS ET AL. 'Gellan gum, production of a bacterial exopolysaccharide microorganism screening on gelrite-containing culture medium and Sphingomonas paucimobilis new strain isolation'
- D. LOBAS ET AL.,: "Structure and physical properties of the extracellular polysaccharide PS-P4 produced by Sphingomonas paucimobilis P4 (DSM6418)", CARBOHYDRATE RESEARCH, AMSTERDAM, 1994, Band 251, Nr. , Seiten 303 - 313

## Beschreibung

Es ist erwünscht, den Stand der Technik, der die biotechnologische Herstellung von Polysacchariden betrifft, zu verbessern.

Gellan beispielsweise ist ein mikrobielles, anionisches Heteropolysaccharid aus Tetrasaccharid-Grundeinheiten, das von Auromonas elodea ATCC 31461 gebildet wird. Natives Gellan ist mit Acetyl- und L-Glycerinsäureresten substituiert. Diese Substituenten lassen sich durch eine Alkalibehandlung leicht abspalten. Das dadurch erhältliche unsubstituierte Gellan (im folgenden Gellan/u) ist ein technisch interessantes Produkt, das die physikalische Eigenschaft aufweist, in wässerigen Lösungen thermoreversibel Gele zu bilden. Gellan/u wird bereits als Agar-Ersatz vertrieben (Gelrite von Kelco, Rahway/USA). Für die Lebensmittelindustrie ist ein weites Feld von Applikationen denkbar. Eine Zulassung als Lebensmittelzusatz in den USA und in Europa ist bereits beantragt worden [1]. In Japan ist Gellan bereits seit 1988 als Lebensmittelzusatz zugelassen [1].

Bakterienspezies, bei denen die Bildung eines Polysaccharids, wie beispielsweise Gellan, zu beobachten ist, sind bekannt. Die Schwierigkeit, derartige Spezies zur Produktion von Polysacchariden heranzuziehen, besteht darin, daß nicht alle Stämme, Klone oder Zellen dieser Spezies das gewünschte Polysaccharid effizient extrazellulär bilden.

EP-A-0 012 552 beschreibt ein Verfahren zur Herstellung des Polysaccharids S-60 unter Verwendung des Pseudomonas-Stammes (Auromonas elodea) ATCC 31461. Pure & Applied Chemistry, 56 (1984) 879-892 und J. Applied Bacteriol., 62 (1987) 147-150 beschreiben ebenfalls die Herstellung von extrazellulären Polysacchariden (Gellan) mit Hilfe von Zellen (Pseudomonas elodea und Pseudomonas paucimobilis). In EP-A-0 353 145, DE-A-3 445 302 und EP-A-0 232 582 werden Zellen beschrieben, die auch extrazelluläre Polysaccharide bilden.

Es wurde nun überraschenderweise festgestellt, daß Zellen, die Gellan extrazellulär produzieren, nicht in eine Gellanmatrix einsinken, während Zellen, die kein Gellan extrazellulär produzieren, in der Gellanmatrix untergehen. Ausgehend von der genannten Beobachtung wurde nun ein Verfahren entwickelt, mit dem sich Zellen und Klone isolieren lassen, die ein gewünschtes Polysaccharid, wie Gellan, effizient extrazellulär produzieren.

Zur Lösung der der Erfindung zugrundeliegenden Aufgabe wird gemäß einer Ausführungsform der Erfindung ein Verfahren zur Isolierung von Zellen einer Bakterienspezies vorgeschlagen, bei der die Bildung eines Polysaccharids beobachtet wird, wobei die zu isolierenden Zellen das Polysaccharid extrazellulär bilden, und das Verfahren dadurch gekennzeichnet ist, daß man
(a) einen Nährboden mit unsubstituiertem Gellan (Gellan/u) als Gelbildner verwendet,
(b) die Zellen der Spezies, von denen man ausgeht, durch Verdünnungsausstrich vereinzelt und
(c) die Zellen oder Klone isoliert, die nicht in die Nährbodenmatrix einsinken.

Bei dem erfindungsgemäßen Verfahren kann man von Zellen einer Bakterienspezies ausgehen, bei der die Bildung eines Polysaccharids beobachtet wird, und Zellen oder Klone isolieren, die das Polysaccharid in effizienterer Weise extrazellulär bilden als die Ausgangskultur.

Auch kann man bei dem erfindungsgemäßen Verfahren von Zellen einer Bakterienspezies ausgehen, bei der die Bildung von Gellan beobachtet wird, und Zellen oder Klone isolieren, die Gellan extrazellulär bilden.

Man kann dabei von Zellen einer Spezies der Gattung Auromonas oder Sphingomonas, insbesondere von Zellen der Spezies Auromonas elodea oder Sphingomonas paucimobilis, beispielsweise von Zellen von Auromonas elodea ATCC 31461 ausgehen. Aus dieser Kultur wurde nach dem erfindungsgemäßen Verfahren Sphingomonas paucimobilis DSM 6314 erhalten, ein effizienter Gellanbildner.

Ferner kann man bei dem erfindungsgemäßen Verfahren von Zellen einer Bakterienspezies ausgehen, bei der die Bildung des Polysaccharids P4 beobachtet wird, und Zellen oder Klone isolieren, die P4 extrazellulär bilden.

Das Exopolysaccharid P4 ist nach ersten Analysen (Zuckersequenzierung) aus zwei Teilen Glucose und einem Teil Rhamnose aufgebaut. In wäßriger Lösung zeigt P4 viskositätserhöhende Eigenschaften. Die Viskosität von wäßrigen P4-Lösungen bleibt im Bereich von pH 2 bis pH 10 und bei Temperaturen bis etwa 80°C stabil. Mit steigender Salinität fällt die Viskosität wäßriger P4-Lösungen nur langsam ab. Nach Erhitzen im alkalischen Medium bildet P4 feste Gele.

Man kann dabei von Zellen einer Spezies der Gattung Pseudomonas oder Sphingomonas, insbesondere von Zellen der Spezies Pseudomonas paucimobilis oder Sphingomonas paucimobilis, insbesondere von Zellen von Pseudomonas paucimobilis NCIMB 11 942 ausgehen. Aus dieser Kultur wurde nach dem erfindungsgemäßen Verfahren Sphingomonas paucimobilis DSM 6 418 (im folgenden auch P4) erhalten, ein effizienter P4-Bildner.

Gemäß einer weiteren Ausführungsform der Erfindung wird Sphingomonas paucimobilis DSM 6314 vorgesehen. Es handelt sich um einen Stamm, der nach dem erfindungsgemäßen Verfahren erhalten worden ist und in effizienter Weise Gellan extrazellulär bildet.

Ferner wird gemäß einer weiteren Ausführungsform der Erfindung Sphingomonas paucimobilis DSM 6 418 vorgesehen. Es handelt sich um einen Stamm, der nach dem erfindungsgemäßen Verfahren erhalten worden ist und in effizienter Weise P4 extrazellulär bildet.

Schließlich wird gemäß einer weiteren Ausführungsform der Erfindung ein Verfahren zur chargenweisen oder kontinuierlichen Herstellung des Polysacchardis P4 vorgesehen, das dadurch gekennzeichnet ist, daß man das Polysaccharid mit Hilfe von Zellen oder Klonen von DSM 6 418 herstellt.

Durch eine neu entwickelte Screeningtechnik konnten aus einer Kultur des Stammes (ATCC 31461) Auromonas elodea [2], (vor 1987 Pseudomonas elodea [3]), zwei Stämme isoliert werden, deren Charakterisierung durch die Deutsche Sammlung von Mikroorganismen und Zellkulturen (DSM), Braunschweig, eine Einordnung unter dem systematischen Namen Sphingomonas paucimobilis ergab. Es handelt sich um neue bisher unbekannte Mikroorganismen, die sich in mehreren Merkmalen von denen des Stammes (ATCC 31461) unterscheiden. In der Literatur wird die Kultur (ATCC 31461) als pleomorph bezeichnet [3]. Kang et. al. [3] erwähnen, daß nur bei Verwendung einer frisch ausplattierten Kultur für das Inocculum ein gutes Wachstum und eine gute Produktion von Gellan resultiert [4]. Dies ist möglicherweise dadurch zu erklären, daß sich mit zunehmenden Alter der Kultur nichtproduzierende Varianten durchsetzen. Von den zwei in Reinkultur isolierten Varianten des Stammes Sphingomonas paucimobilis produziert nur die Variante E2, nicht aber die Variante E1 extrazelluläres Polysaccharid. Die technisch interessante Variante Sphingomonas paucimobilis E2 wurde als Lyophilisiat bei der DSM unter der Nummer 6314 hinterlegt. Auch Mischkulturen der Varianten E1 und E2 verlieren relativ schnell ihr Potential zur Produktion des Polysaccharids Gellan. Die Folge aus der Unkenntnis dieser Sachverhalte sind höchst uneinheitliche Fermentationsergebnisse, bedingt durch ein unkontrollierbares Impfgut.

Als ein weiterer Erfolg der neu entwickelten Screeningtechnik konnten aus einer Kultur des Stammes Pseudomonas paucimobilis (NCIMB 11942) [5] mehrere verschiedene Stämme isoliert werden. Die Charakterisierung der in Reinkultur erhaltenen Variante P4 durch die Deutsche Sammlung von Mikroorganismen und Zellkulturen (DSM), Braunschweig, ergab ebenfalls eine Einordnung unter dem systematischen Namen Sphingomonas paucimobilis. Es handelt sich um eine neue bisher unbekannte Mikroorganismenspezies, die sich in mehreren Merkmalen von denen des Stammes Pseudomonas paucimobilis (NCIMB 11942) unterscheiden. Von den isolierten Varianten aus der Kultur Pseudomonas paucimobilis (NCIMB 11942) produziert nur die Variante Sphingomonas paucimobilis P4 das extrazelluläres Polysaccharid P4. Die technisch interessante Variante Sphingomonas paucimobilis P4 wurde als Lyophilisiat bei der DSM unter der Nummer 6418 hinterlegt. Mischkulturen der Varianten verlieren relativ schnell ihr Potential zur Produktion des Polysaccharids P4.

Sphingomonas paucimobilis E2 (DSM 6314) produziert bei aerober Fermentation aus verschiedenartigen Kohlenstoffquellen wie z.B. Glucose, mit Ammoniumnitrat oder anderen Stickstoffquellen, bei Anwesenheit von Mineralsalzen und Spurenelementen sowie mit oder ohne komplexe Nährmedien, das Exopolysaccharid Gellan. Die Fermentationszeit zur Umsetzung von 30 kg/m³ Glucose liegt unter 46,5 Stunden. Die Fermentationsbrühe zeigt thixotropes Verhalten. Nach vorheriger Scherung lassen sich die Fließeigenschaften der pseudoplastischen Gellan-Lösungen mit der Beziehung nach Ostwald und de Waele beschreiben. Während der Fermentation steigt die Viskosität erheblich an. Am Ende der Fermentation werden Konsistenzfaktoren über 20000 mPa sⁿ erreicht. Der Fließindex n fällt dabei auf den Wert 0,11 . Die Ausbeute an reinem Gellan beträgt mehr als 28 % bezogen auf die eingesetzte Kohlenstoffquelle. Hierin ist eine deutliche Steigerung der Produktausbeute gegenüber dem herkömmlichen Verfahren zu sehen. Kang et. al. [3] erhalten 50 Gew. % Polysaccharid und Biomasse bezogen auf die eingesetzte Kohlenstoffquelle, wobei davon etwa 50 Gew.% aus Protein und unlöslichen (Zell-) Bestandteilen bestehen, d.h. die Ausbeute an reinem Gellan liegt nach diesen Angaben bei 25 Gew.% bezogen auf die eingesetzte Kohlenstoffquelle [4,6]. Da hier Abweichnugen durch unterschiedliche analytische Methoden möglich sind, wurden eigene Versuche auch mit dem Stamm Auromonas elodea (ATCC 31461) durchgeführt. In diesen Vergleichsfermentationen wurde beobachtet, daß die tatsächliche Ausbeute an reinem, proteinfreiem Gellan nach dem Fermentationsverfahren von Kang mit Auromonas elodea (ATCC 31461) erheblich geringer ist und etwa bei ca. 6-10 % liegt.

### Isolierung der Kulturen

Wenn man eine Kultur von Auromonas elodea (ATCC 31461) bzw. Pseudomonas paucimobilis (NCIMB 11942) auf festen Nährböden mit Gellan/u statt Agar ausplattiert, zeigt sich nach ca. 48 h Inkubation bei 30 °C, daß die Kolonien in die Matrix einsinken. Versuche ohne Kohlenstoffquelle zeigten kein Wachstum, so daß offenbar Gellan/u nicht als Substrat verwertet wird. Das Gel wird durch die Mikroorganismen jedoch verflüssigt. Bei Verdünnungsreihen von Auromonas elodea (ATCC 31461) bzw. Pseudomonas paucimobilis (NCIMB 11942) in physiologischer Natriumchloridlösung, und anschließendes Ausplattieren auf Yeast/Malt-Gellan/u Platten zeigte sich, daß einige Kolonien nicht in die Matrix des Gellan/u einsanken. Diese Kulturen erwiesen sich als besonders gut für die Polysaccharidproduktion geeignet. Durch mehrfaches Überimpfen und Wiederholen dieses Aufreinigungsschrittes erhielten wir die Reinkulturen der Stämme Sphingomonas paucimobilis E2 (DSM 6314) bzw. Sphingomonas paucimobilis P4 (DSM 6418). Die nicht produzierenden Varianten wurde durch Vereinzelung aus einsinkenden Kolonien erhalten.

Um bei der Fermentation eine möglichst hohe Produktausbeute zu erreichen, muß eine Kontamination durch die nicht produzierenden Varianten ausgeschlossen werden. Die besten Ergebnisse erzielt man, indem man die erste Vorkultur für die Fermentation aus einer Vedünnungsreihe animpft.

### Stammhaltung und Anzuchttechnik

Die Stammhaltung von Sphingomonas paucimobilis E2 (DSM 6314) kann routinemäßig auf Yeat/Malt-Platten mit Agar oder Gellan/u als Gelbildner erfolgen, auf denen gutes Wachstum zu verzeichnen ist. Die Stammhaltung von Sphingomonas paucimobilis P4 (DSM 6418) kann routinemäßig auf Czapek Dox-Platten oder auf Yeat/Malt-Platten erfolgen; auf beiden Medien ist gutes Wachstum zu verzeichnen. Die Selektion der produzierenden Stammvarianten erfolgt auf Yeast/Malt-Gellan/u-Platten. Die Platten werden in der Regel 1-2 Tage bei 30 °C inkubiert.

Die Kulturen sind ca. 1-2 Wochen bei 4 °C lagerfähig, ohne daß die Mikroorganismen einer Aktivitätsminderung unterliegen. Als eine andere Art der Konservierung ist es z.B. möglich, die Kulturen ohne Vorbehandlung bei -20 °C tiefzukühlen. Vor einer Verwendung sollten diese Kulturen jedoch einmal überimpft werden. In der DSM wurden die Kulturen als Lyophilisiate hinterlegt.

Zur ersten Vorkultur wurden Kolben mit 25 ml des Fermentationsmediums verwendet. Jede folgende Stufe wurde mit jeweils 10 % ihres Volumens an Inoculum aus der vorhergehenden Stufe beimpft. Die Inkubation erfolgte bei 30 °C auf einem Schüttler bei 180 rpm für jeweils 24 h.

Alle Bestandteile des Nährmediums bis auf die Glucose wurden zusammen bei 121 °C sterilisiert. Die Glucose wurde separat gelöst und sterilisiert, um ein Karamelisierung zu vermeiden. Die Glucoselösung und die Lösung mit den anderen Komponenten wurden unter einer Cleanbench zur Nährlösung vereinigt. Das für die submersen Kultivierungen (Schüttelkolben und Fermenter) verwendete Nährmedium nach Kang [3] hat folgende Zusammensetzung:

| | |
|---|---|
| 3,3 % | Glucose H₂O |
| 0,01 % | MgSO₄ 7 H₂O |
| 0,09 % | NH₄NO₃ |
| 0,05 % | Promosoy (Central Soya Overseas B.V., Chem. Div., Rotterdam) |
| 1 ml | Spurenelementkonzentrat Lösung |
| 5 ml | Kaliumphosphatpuffer pH 7 (1 molar) |

Die Spurenelementkonzentratlösung besitzt folgende Zusammensetzung:

| | |
|---|---|
| Manganchlorid 4 H₂O | 1,800 g/l |
| Eisen II sulfat 7 H₂O | 2,488 g/l |
| Borsäure | 0,285 g/l |
| Kupfersulfat 5 H₂O | 0,052 g/l |
| Zinkchlorid | 0,021 g/l |
| Kobaltchlorid 6 H₂O | 0,074 g/l |
| Magnesiummolybdat | 0,023 g/l |
| Natriumnitrat 2 H₂O | 2,1 g/l |

Neben der aufgeführten wurden auch andere Mediumszusammensetzungen erfolgreich zur Gellanproduktion und zur Herstellung des Polysaccharids P4 verwendet.

### Charakterisierung des Stammes Sphingomonas paucimobilis E2 (DSM 6314)

### Morphologie

Bei den Bakterien Sphingomonas paucimobilis E2 (DSM 6314) handelt es sich um Gram-negative, stäbchenförmige Zellen von 0,6-0,8 µm Breite und 1,5-4,0 µm Länge. Die Zellen in frischen Kulturen sind gut beweglich. Mit zunehmendem Alter der Kulturen wird die Beweglichkeit durch das gebildete Polysaccharid stark eingeschränkt. Die Mikroorganismen wachsen nur aerob.

Die Bakterien bilden auf Y/M-Agar nach 48 h Inkubation Kolonien mit einem Durchmesser von 2-3 mm. Die Kolonien sind durch ein nicht diffundierendes Pigment gelb gefärbt. Die Stammvariante E1 ist intensiv gelb und erzeugt auf festen Nährböden harte nicht viskose Kolonien. Die Variante E2 erscheint auf festen Nährböden etwas dunkler gelb als die Variante E1. Wegen des gebildeten Polysaccharides sind die Kolonien der Variante E2 viskos. Über längere Zeit inkubierte Platten von E2 zeigen transparente Kolonien mit eingelagerten gelben Zellanhäufungen, wobei augenscheinlich Polysaccharid in erheblichen Mengen ausgeschieden wird.

### Physiologie und biochemische Eigenschaften

Die biochemische Charakerisierung der Kulturen erfolgte in unserem Auftrag durch die Deutsche Sammlung von Mikroorganismen und Zellkulturen (DSM), Braunschweig. Diese Untersuchungen ergaben, daß es sich bei Sphingomonas paucimobilis E2 (DSM 6314) um eine neue bisher nicht bekannte Spezies handelt. Auromonas elodea (ATCC 31461) zeigte bei einer parallelen Untersuchung deutliche Unterschiede. Die Bakterienkultur Sphingomonas paucimobilis E2 (DSM 6314) besitzt folgende physiologische und biochemische Eigenschaften:

**Tabelle 1:**

| Charakterisierung von Sphingomonas paucimobilis E2 (DSM 6314) | | | |
|---|---|---|---|
| Test/Eigenschaft | | | |
| | | ADH | - |
| | | | |
| | | ODC | - |
| | | | |
| Lyse durch 3% KOH | + | VP | - |
| | | | |
| Aminopeptidase (Cerny) | + | Indol | - |
| | | | |
| Sporen | - | NO₂ aus NO₃ | - |
| | | | |
| Oxidase | + | Denitrification | - |
| | | | |
| Catalase | + | Phenylalanindesaminase | - |
| | | | |
| Wachstum | | Levan aus Saccharose | - |
| anaerob | - | | |
| 37/40 °C | +/- | Lecithinase | - |
| pH 5,6 | - | | |
| Mac-Conkey-Agar | - | Urease | - |
| SS-Agar | - | | |
| Cetrimid-Agar | - | Hydrolyse von | |
| | | Stärke | + |
| Pigmente | gelb | Gelatine | - |
| nicht diffundierend | + | Casein | - |
| diffundierend | - | DNA | + |
| fluoreszierend | - | Tween 80 | + |
| Pyocyanin | - | Äsculin | + |
| | | | |
| Säure aus (OF-Test) | | Tyrosin-Abbau | + |
| Glucose aerob | - | | |
| Glucose anaerob | - | Substratverwertung | |
| | | Acetat | + |
| Gas aus Glucose | - | Adipat | - |
| | | Caprat | - |
| Säure aus (ASS) | | Citrat | - |
| Glucose | + | Glycolat | - |
| Fructose | + | Lactat | - |
| Xylose | + | Lävulinat | - |
| Arabinose | + | Malat | + |
| Fucose | - | Malonat | - |
| Maltose | + | Phenylacetat | - |
| Mannose | + | Suberat | - |
| Lactose | + | L-Arabinose | + |
| Saccharose | + | Fructose | + |
| Trehalose | + | Glucose | + |
| Cellobiose | + | Mannose | + |
| Rhamnose | - | Xylose | + |
| Raffinose | + | Mannitol | - |
| Melezitose | + | Gluconat | - |
| Melibiose | + | 2-Ketogluconat | - |
| Aldonitol | - | N-Acetylglucosamin | + |
| Dulcitol | - | L-Histidin | - |
| Inositol | - | L-Serin | - |
| Mannitol | - | Hydroxybutyrat | - |
| Sorbitol | - | | |
| N-Acetylglucosamin | + | | |
| Glycerol | - | | |

### Charakterisierung des Stammes Sphingomonas paucimobilis P4 (DSM 6418)

### Morphologie

Bei den Bakterien Sphingomonas paucimobilis P4 (DSM 6418) handelt es sich um Gram-negative, stäbchenförmige Zellen von 0,6-0,8 *µ*m Breite und 1,5-4,0 µm Länge. Die Zellen in frischen Kulturen sind gut beweglich. Mit zunehmendem Alter der Kulturen wird die Beweglichkeit durch das gebildete Polysaccharid stark eingeschränkt. Die Mikroorganismen wachsen nur aerob.

Die Bakterien bilden auf Y/M-Agar nach 48 h Inkubation Kolonien mit einem Durchmesser von 1-2 mm. Die Kolonien sind durch ein nicht diffundierendes Pigment gelb gefärbt. Die Stammvariante P4 ist intensiv gelb und erzeugt auf festen Nährböden harte nicht viskose Kolonien. Auf Czapek Dox Agar erscheinen die Kolonien leuchtend gelb und leicht transparent, die Kolonien auf diesem Medium sind extrem hart und zäh.

### Physiologie und biochemische Eigenschaften

Die biochemische Charakerisierung der Kulturen erfolgte in unserem Auftrag durch die Deutsche Sammlung von Mikroorganismen und Zellkulturen (DSM), Braunschweig. Diese Untersuchungen ergaben, daß es sich bei Sphingomonas paucimobilis P4 (DSM 6418) um eine neue bisher nicht bekannte Spezies handelt. Pseudomonas paucimobilis (NCIMB 11942) zeigte bei einer parallelen Untersuchung deutliche Unterschiede. Die Bakterienkultur Sphingomonas paucimobilis P4 (DSM 6418) besitzt folgende physiologische und biochemische Eigenschaften:

**Tabelle 2:**

| Charakterisierung von Sphingomonas paucimobilis P4 (DSM 6418) | | | |
|---|---|---|---|
| Test/Eigenschaft | | | |
| | | ADH | - |
| | | | |
| | | ODC | - |
| | | | |
| Lyse durch 3% KOH | + | VP | - |
| | | | |
| Aminopeptidase (Cerny) | + | Indol | - |
| | | | |
| Sporen | - | NO₂ aus NO₃ | - |
| | | | |
| Oxidase | + | Denitrification | - |
| | | | |
| Catalase | + | Phenylalanindesaminase | - |
| | | | |
| Wachstum | | Levan aus Saccharose | - |
| anaerob | - | | |
| 37/40 °C | +/- | Lecithinase | - |
| pH 5.6 | - | | |
| Mac-Conkey-Agar | - | Urease | - |
| SS-Agar | - | | |
| Cetrimid-Agar | - | Hydrolyse von | |
| | | Stärke | + |
| Pigmente | gelb | Gelatine | - |
| nicht diffundierend | + | Casein | - |
| diffundierend | - | DNA | + |
| fluoreszierend | - | Tween 80 | + |
| Pyocyanin | - | Äsculin | + |
| | | | |
| Säure aus (OF-Test) | | Tyrosin-Abbau | + |
| Glucose aerob | - | | |
| Glucose anaerob | - | Substratverwertung | |
| | | Acetat | + |
| Gas aus Glucose | - | Adipat | - |
| | | Caprat | - |
| Säure aus (ASS) | | Citrat | - |
| Glucose | + | Glycolat | - |
| Fructose | + | Lactat | - |
| Xylose | + | Lävulinat | - |
| Arabinose | + | Malat | + |
| Fucose | + | Malonat | - |
| Maltose | + | Phenylacetat | - |
| Mannose | + | Suberat | - |
| Lactose | + | L-Arabinose | + |
| Saccharose | + | Fructose | + |
| Trehalose | + | Glucose | + |
| Cellobiose | + | Mannose | + |
| Rhamnose | - | Xylose | + |
| Raffinose | + | Mannitol | - |
| Melezitose | + | Gluconat | - |
| Melibiose | + | 2-Ketogluconat | - |
| Aldonitol | - | N-Acetylglucosamin | + |
| Dulcitol | - | L-Histidin | - |
| Inositol | - | L-Serin | + |
| Mannitol | - | Hydroxybutyrat | - |
| Sorbitol | - | | |
| N-Acetylglucosamin | - | | |
| Glycerol | - | | |

### Vergleich von Sphingomonas paucimobilis E2 und P4 mit Auromonas elodea (ATCC 31461) und Pseudomonas paucimobilis (NCIMB 11942)

Die Stämme Sphingomonas paucimobilis E2 und P4 unterscheiden sich in wesentlichen Merkmalen von den Ausgangskulturen Auromonas elodea (ATCC 31461) und Pseudomonas paucimobilis (NCIMB 11942). In der folgenden Tabelle sind diejenigen Merkmale aufgeführt, bei denen sich Unterschiede ergaben.

**Tabelle 3:**

| Vergleich der Unterschiede der Varianten E2 und P4 von Sphingomonas paucimobilis und Auromonas elodea (ATCC 31461) und Pseudomonas paucimobilis (NCIMB 11942) | | | | |
|---|---|---|---|---|
| Eigenschaften des Stammes | DSM 6314 (E2) | ATCC 31461 | DSM 6418 (P4) | NCIMB 11942 |
| Zellform: Länge µm | 1,5-4,0 | 1,5-5,0 | 1,5-4,0 | 1,5-4,0 |
| Wachstum bei 37 °C: | + | - | + | + |
| Säure aus (ASS): | | | | |
| Fucose | - | - | + | + |
| Melezitose | + | - | + | - |
| N-Acetylglucosamin | + | + | - | + |
| Verflüssigung von Gellan/u-Gel: | - | + | - | + |
| Substratverwertung: | | | | |
| L-Arabinose | + | w | + | w |
| 2-Ketogluconat | - | + | - | + |
| L-Serin | - | - | + | + |

### 1. Beispiel (Vergleichsfermentationen)

Im folgenden sollen typische Satzfermentationen der Stämme Sphingomonas paucimobilis E2 (DSM 6314) und Auromonas elodea (ATCC 31461) miteinander verglichen werden. Beide Fermentationen wurden unter gleichen Bedingungen in einem Biostat E Fermenter (B. Braun, Melsungen) im Maßstab von 10 l ausgeführt. Als erste Vorkultur wurden vier Kolben mit jeweils 25 ml des oben angegebenen Kulturmediums von einer 3 Tage alten Yeast/Malt-Gellan/u Platte beimpft. Die zweite Vorkultur (vier Kolben mit jeweils 250 ml) wurde nach 24 h mit der Gesamtmenge der ersten Vorstufe angeimpft. Diese zweite Vorstufe diente nach weiteren 24 h als Inoculum für den Fermenter.

Der Fermenter wurde mit 0,33 vvm Luft begast. Die Temperatur betrug 30 °C. Um stagnante Zonen im Fermenter zu vermeiden und eine gute Durchmischung zu gewährleisten, wurde die Kulturlösung mit einem vierstufigen Intermig-Rührer (Durchmesser 63 % des Behälterdurchmessers) mit 800 Upm gerührt. Im Kulturmedium wurde ein pH-Wert von 7 eingestellt und mittels 1 n Natronlauge und 1 n Phosphorsäure über eine Regelung konstant gehalten.

Die Gellankonzentration am Ende der Fermentation war bei Sphingomonas paucimobilis E2 (DSM 6314) etwa um den Faktor 4 größer als bei der Fermentation mit Auromonas elodea (ATCC 31461) (**Abb.1**). Die maximalen Gellankonzentrationen betrugen 8 kg/m³ für Sphingomonas paucimobilis E2 (DSM 6314) und 2 kg/m³ für Auromonas elodea (ATCC 31461). Für die Raum-Zeit-Ausbeuten dieser Fermentationen ergaben sich 0,172 kg/m³ n für Sphingomonas paucimobilis E2 (DSM 6314), und 0,04 kg/m³ h für Auromonas elodea (ATCC 31461).

Die Bestimmung des Gesamtgehaltes an Gellan und Biomasse erfolgte durch Zusatz von zwei Volumenteilen Isopropanol, Abzentrifugieren und Trocknen (48 Stunden bei 80 °C). Zur Bestimmung des Biotrockenmasse-Gehaltes wurden die Proben mit dest. Wasser verdünnt, die Zellen abzentrifugiert, der Überstand dekantiert, das Sediment mit Wasser aufgenommen, und nach erneutem Zentrifugieren und Dekantieren das Sediment getrocknet. Die Ermittlung der Gellankonzentration erfolgte durch Differenzbildung aus den Ergebnissen dieser Bestimmungen.

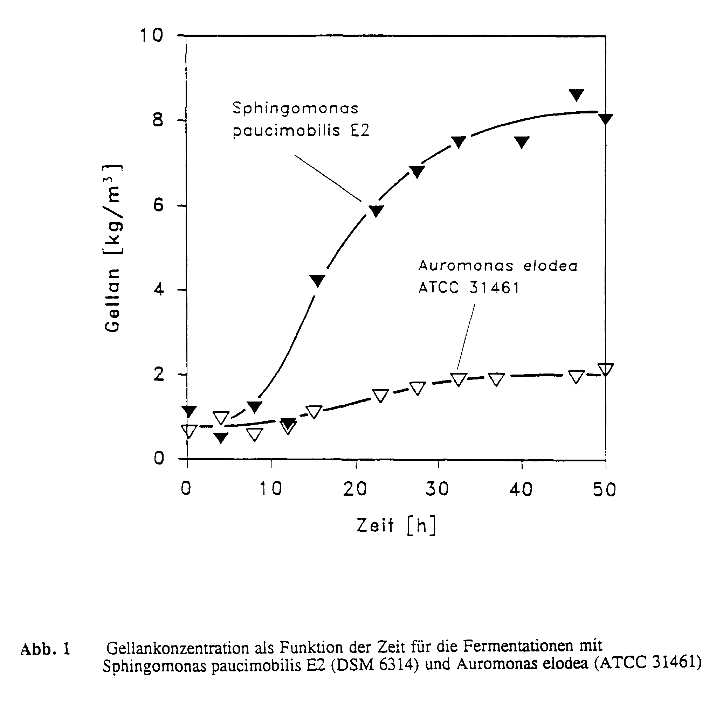

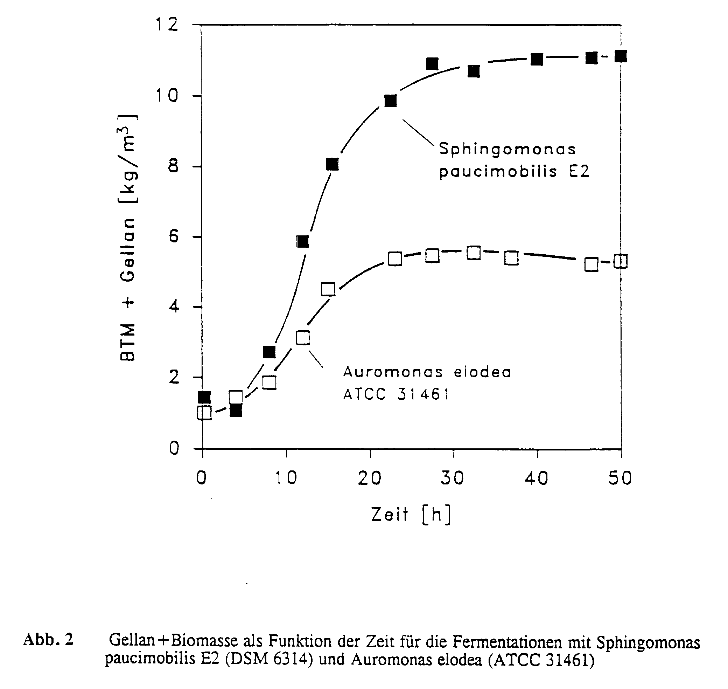

Bedingt durch die hohe Viskosität der Fermentationsbrühe bei Sphingomonas paucimobilis E2 (DSM 6314) war es notwendig, die Proben vor der Zentrifugation stärker zu verdünnen. Aus diesem Grund streuen die Meßwerte weiter als bei der Fermentation mit Auromonas elodea (ATCC 31461). Die geringsten Fehler entstehen bei der Bestimmung der Summe von Gellan- und Biomassegehalt (von Kang [4] als natives Gellan bezeichnet) aus der unverdünnten Fermentationsbrühe (**Abb. 2**).

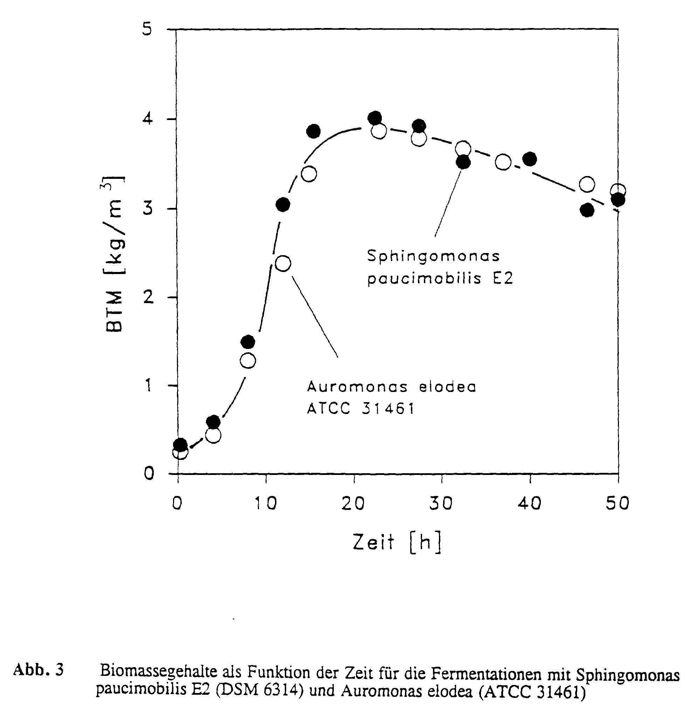

In beiden Fermentationen wurden in etwa gleiche Biotrockenmasse-Gehalte (BTM) erreicht. Der Biomassegehalt erreichte Maximalwerte von etwa 4 kg/m³ am Ende der Wachstumsphase. Danach starben die Mikroorganismen teilweise ab; Der Biomassegehalt sank (Abb. 3). Bei der Fermentation mit Sphingomonas paucimobilis E2 (DSM 6314) war nach 46,5 die Glucose vollständig verbraucht, und damit die Fermentation beendet. Bei Auromonas elodea (ATCC 31461) waren nach 46,5 h noch 2,3 kg/m³ Glucose vorhanden, nach 50 h noch 0,42 kg/m³.

### 2. Beispiel

Die Fermentation des Stammes Sphingomonas paucimobilis P4 (DSM 6418) verlief im wesentlichen ähnlich den vorher beschriebenen Fermentationen. Die Fermentation wurde unter gleichen Bedingungen in einem Biostat E Fermenter (B. Braun, Melsungen) im Maßstab von 10 1 ausgeführt. Als erste Vorkultur wurden vier Kolben mit jeweils 25 ml des oben angegebenen Kulturmediums von einer 3 Tage alten Capek Dox Gellan/u Platte beimpft. Die zweite Vorkultur (vier Kolben mit jeweils 250 ml) wurde nach 24 h mit der Gesamtmenge der ersten Vorstufe angeimpft. Diese zweite Vorstufe diente nach weiteren 24 h als Inoculum für den Fermenter.

Der Fermenter wurde mit 0,33 vvm Luft begast. Die Temperatur betrug 30 °C. Um stagnante Zonen im Fermenter zu vermeiden und eine gute Durchmischung zu gewährleisten, wurde die Kulturlösung mit einem vierstufigen Intermig-Rührer (Durchmesser 63 % des Behälterdurchmessers) mit 800 Upm gerührt. Im Kulturmedium wurde ein pH-Wert von 7 eingestellt und mittels l Natronlauge und l n Phosphorsäure über eine Regelung konstant gehalten.

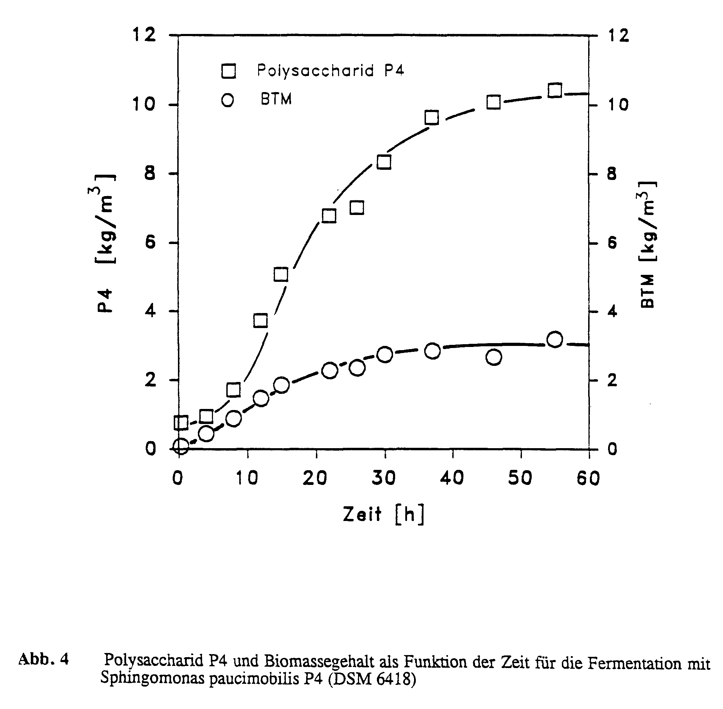

Die Bestimmung des Gesamtgehaltes an P4 und Biomasse erfolgte durch Zusatz von zwei Volumenteilen Isopropanol, Abzentrifugieren, Dekantieren und Trocknen (48 Stunden bei 80 °C). Zur Bestimmung des Biotrockenmasse-Gehaltes wurden die Proben mit Dimethylsulfoxid verdünnt, die Zellen abzentrifugiert, der Überstand dekantiert, das Sediment mit Wasser aufgenommen, und nach erneuten Zentrifugieren und Dekantieren das Sediment getrocknet. Die Ermittlung der Polysaccharidkonzentration erfolgte durch Differenzbildung aus den Ergebnissen dieser Bestimmungen. Die P4-Konzentration am Ende der Fermentation betrug ca. 10 kg/m³ (**Abb. 4**).

### Drittes Beispie

Es wurden folgende Stamme eingesetzt:
*Leuconostoc mesenteroides* DSM 20187
*Enterobacter sakazakll* DSM 4485
*Xanthomonas campestris* NRRL 8-1459 S4L-II
*Aureobasidium pullulans* DSM 2404

Als Medium wurden entsprechend den Wachstumsanforderungen der Stämme die von der DSM vorgeschlagenen Standardmedien benutzt, die dem DSM-Katalog "Catalogue of Strains 1989", 4. Auflage, zu entnehmen sind. In Abweichung vom DSM-Katalog und in Übereinstimmung mit dem erfindungsgemäßen Verfahren wurde jedoch für alle Medien Gellan/u an Stelle von Agar verwendet. Der Ausstrich der Verdünnungsreihen, die Inkubation und die Weiterbehandlung erfolgten in der weise, wie sie für das erfindungsgemaße Verfahren für die Gellan und P4 bildenden Spezies beschrieoen wurden.

**Tabelle 4**

| Verwendete Medien | | |
|---|---|---|
| Stamm | Medium | DSM Catalogue of Strains 1989 |
| *Leuconostoc mesenteroides* (DSM 20187) | MRS | Medium 11, S. 279 |
| *Enterobacter sakazakii* (DSM 4485) | Nutrient | Medium 1, S. 279 |
| *Xanthomonas campestris* (NRRL B-1459 S4L-II) | Yeast/Malt | siehe Stammhaltung und Aufzuchttechnik |
| *Aureobasidium pullulans* (DSM 2404) | Potato Glucose | Medium 129, S. 290 |

Die Stämme Enterobacter sakazakii DSM 4485, Xanthomonas campestris NRRL B-1459 S4L-II, Leuconostoc mesenteroides DSM 20187 und Aureobasidium pullulans DSM 2404 zeigten innerhalb der eingesetzten Zellpopulationen keine einsinkenden Zellen, vielmehr wuchsen alle Zellen auf der Oberfläche der Gellan/u-Platten. Daraus ergibt sich, daß es sich bei den eingesetzten Populationen um Reinkulturen mit hohem Potential zur Polysaccharidbildung handelt.

### Literatur

[1] V.J. Morris, Food Biotechnoiogy 4 (1990) Nr. 1, S. 45-57.
[2] R. Moorhouse, Structure/property relationships of a family of microbial ploysaccharides, in: Industrial Polysaccharides: Genetic Engeneering, Structure/property Relations and Applications. Hrsg. M. Yalpani, Bd. 3, Elsevier Science Publishers, Amsterdam 1987, S. 187-205
[3] K.S. Kang, G.T. Veeder, P.J. Mirrasoul, T. Kaneko und I.W. Cottrell, Applied and Environmental Microbiology 43 (1982) Nr. 5, S. 1086-1091
[4] K.S. Kang, G.T. Colegrove und G.T. Veeder, Deacetylated Polysaccharide S-60, US-Patent 4,326,052, 1982
[5] A. Anson, P.J. Fisher. A.F.D. Kennedy und I.W. Sutherland, Journal of Applied Bacteriology (1987) Nr. 62, S. 147-150
[6] K.S. Kang und G.T.Veeder, Polysaccharide S-60 and bacterial fermentation process for its preparation, US-Patent 4,326,053, 1982.

## Patentansprüche

1. Verfahren zur Isolierung von Zellen einer Bakterienspezies, bei der die Bildung eines Polysaccharids beobachtet wird, wobei die zu isolierenden Zellen das Polysaccharid extrazellulär bilden, dadurch ***gekennzeichnet,*** daß man
(a) einen Nährboden mit unsubstituiertem Gellan als Gelbildner verwendet,
(b) die Zellen der Spezies, von denen man ausgeht, durch Verdünnungsausstrich vereinzelt und
(c) Zellen oder Klone isoliert, die nicht in die Nährbodenmatrix einsinken.

2. Verfahren nach Anspruch 1, dadurch ***gekennzeichnet,*** daß man von Zellen einer Bakterienspezies ausgeht, bei der die Bildung von Gellan beobachtet wird, und Zellen oder Klone isoliert, die Gellan extrazellulär bilden.

3. Verfahren nach Anspruch 2, dadurch ***gekennzeichnet,*** daß man von Zellen einer Spezies der Gattung *Auromonas* oder *Sphingomonas*, insbesondere von Zellen der Spezies *Auromonas elodea* oder *Sphingomonas paucimobilis*, insbesondere von Zellen von *Auromonas elodea* ATCC 31461 ausgeht.

4. Verfahren nach Anspruch 1, dadurch ***gekennzeichnet*,** daß man von Zellen einer Bakterienspezies ausgeht, bei der die Bildung des mit Hilfe von DSM 6418 herstellbare Polysaccharids P4 beobachtet wird, und Zellen oder Klone isoliert, die dieses Polysaccharid P4, das aus zwei Teilen Glukose und einem Teil Rhamnose aufgebaut ist, extrazellulär bilden.

5. Verfahren nach Anspruch 4, dadurch ***gekennzeichnet,*** daß man von Zellen einer Spezies der Gattung *Pseudomonas* oder *Sphingomonas,* insbesondere von Zellen der Spezies *Pseudomonas paucimobilis* oder *Sphingomonas paucimobilis*, insbesondere von Zellen von *Pseudomonas paucimobilis* NCIMB 11 942 ausgeht.

6. *Sphingomonas paucimobilis* DSM 6 314.

7. *Sphingomonas paucimobilis* DSM 6 418.

8. Verfahren zur chargenweisen oder kontinuierlichen Herstellung des mit Hilfe von DSM 6418 herstellbaren Polysaccharids P4, das aus zwei Teilen Glukose und einem Teil Rhamnose aufgebaut ist.

## Claims

1. Method for isolating cells of bacterial species in which the production of a polysaccharide is observed, the cells to be isolated producing the polysaccharide extracellularly, characterised in that
(a) a nutrient medium is used which comprises unsubstituted gellan as the gellant,
(b) cells of the starting species are separated by the streak method, and
(c) cells or clones, which do not sink into the nutrientmedium matrix, are isolated.

2. Method according to Claim 1, characterised in that the starting cells are cells of a bacterical species in which the production of gellan is observed, and cells or clones which produce gellan extracellularly are isolated.

3. Method according to Claim 2, characterised in that the starting cells are cells of a species from the genus Auromonas or Spingomonas, in particular cells of the species Auromonas elodea or Sphingomonas paucimobilis, in particular cells of Auromonas elodea ATCC 31461.

4. Method according to Claim 1, characterised in that the starting cells are cells of a bacterial species in which the production of polysaccharide P4 which is producable by means of DSM6 418, is observed, and cells or clones are isolated which produce this polysaccharide P4 extracellularly, whereas the polysaccharide P4 is formed by two parts of glucose and one part rhamnose.

5. Method according to Claim 4, characterised in that the starting cells are cells of a species from the genus Pseudomonas or Sphingomonas, in particular cells of the species Pseudomonas paucimobilis of Sphingomonas paucimobilis, in particular cells of Pseudomonas paucimobilis NCIMB 11 942.

6. Sphingomonas paucimobilis DSM 6 314.

7. Sphingomonas paucimobilis DSM 6 418.

8. Method for the batchwise or continuous production of polysaccharide P4 which is producable by means of DSM 6 418 and is formed by two parts of glucose and one part rhamnose.

## Revendications

1. Procédé pour isoler des cellules d'une espèce bactérienne, dans laquelle la formation d'un polysaccharide est observée, les cellules à isoler formant de façon extracellulaire le polysaccharide, caractérisé en ce que
(a) on utilise un milieu nutritif avec de la gomme gellan non substituée en tant que gélifiant,
(b) on isole les cellules de l'espèce, à partir desquelles l'on part, en les étalant avec dilution et
(c) on isole les cellules ou les clones qui ne s'enfoncent pas dans la matrice du milieu nutritif.

2. Procédé selon la revendication 1, caractérisé en ce que l'on part de cellules d'une espèce bactérienne dans laquelle la formation de gomme gellan est observée, et l'on isole des cellules ou des clones qui forment de la gomme gellan de façon extracellulaire.

3. Procédé selon la revendication 2, caractérisé en ce que l'on part de cellules d'une espèce du genre *Auromonas* ou *Sphingomonas*, en particulier de cellules de l'espèce *Auromonas elodea* ou *Sphingomonas paucimobilis*, en particulier de cellules de *Auromonas elodea* ATCC 31461.

4. Procédé selon la revendication 1, caractérisé en ce que l'on part de cellules d'une espèce bactérienne, dans laquelle la formation du polysaccharide P4 est observée, que l'on peut préparer à l'aide de DSM 6418, et l'on isole des cellules ou des clones qui forment de façon extracellulaire ce polysaccharide P4 qui est constitué de deux parties de glucose et d'une partie de rhamnose.

5. Procédé selon la revendication 4, caractérisé en ce que l'on part de cellules d'une espèce du genre *Pseudomonas* ou *Sphingomonas*, en particulier de cellules de l'espèce *Pseudomonas paucimobilis* ou *Sphingomonas paucimobilis*, en particulier de cellules de *Pseudomonas paucimobilis* NCIMB 11 942.

6. *Sphingomonas paucimobilis*, DSM 6 314.

7. *Sphingomonas paucimobilis*, DSM 6 418.

8. Procédé pour préparer par lots ou en continu le polysaccharide P4 que l'on peut préparer à l'aide de DSM 6 418, qui est constitué de deux parties de glucose et d'une partie de rhamnose.
